# EUROPEAN PATENT APPLICATION

(11) **EP 0 578 867 A1**
(43) Date of publication of application: **19.01.1994**
(21) Application number: 92202130.8
(22) Date of filing: 13.07.1992
(51) Int. Cl.: B09B 3/00, B09B 1/00, C12M 1/107

(54) **Method for processing garbage**

(71) Applicant: Doleh, Zakaria Kalil, Dubai (AE)
(72) Inventor: Doleh, Zakaria Kalil, Dubai (AE)
(74) Representative: Hoijtink, Reinoud

(57) **Abstract**

According to the invention garbage is processed by compressing garbage into a container (1), sealing the container and encapsulating the container with metal wire coated with plastic. The interior of the sealed container is by means of flexible tubes (4,5,6) connected with the environment in order to make use of the gas produced by fermentation of the garbage.

## Description

The invention is related to a method for processing garbage comprising the consecutive method steps:
- separating the garbage in fractions,
- compressing the fractions in a container according to a desired predetermined composition,
- placing the container in a casing consisting of a web of metal wire coated with plastic.

Such a method is known from EPA 91202162.3.

It is the object of the invention to improve said method further.

This is achieved according to the invention by sealing said container with respect to the environment and connecting the container by means of a tube to a device for using fermentation gas.

According to the invention use is made of the fermentation of biological material in the garbage and the gas produced by the fermentation is used in for example a gas burner. To this end at any of the end parts of the container a tube is connected. If the bottom on which the container is placed is uneven, the gas will flow to one of the end parts. However, by providing a tube on both ends the gas can still be used.

It is further proposed to connect a tube to the middle part of the container.

The gas burner can be used for powering an electrical generator.

The invention will be elucidated with the help of the drawings in which:

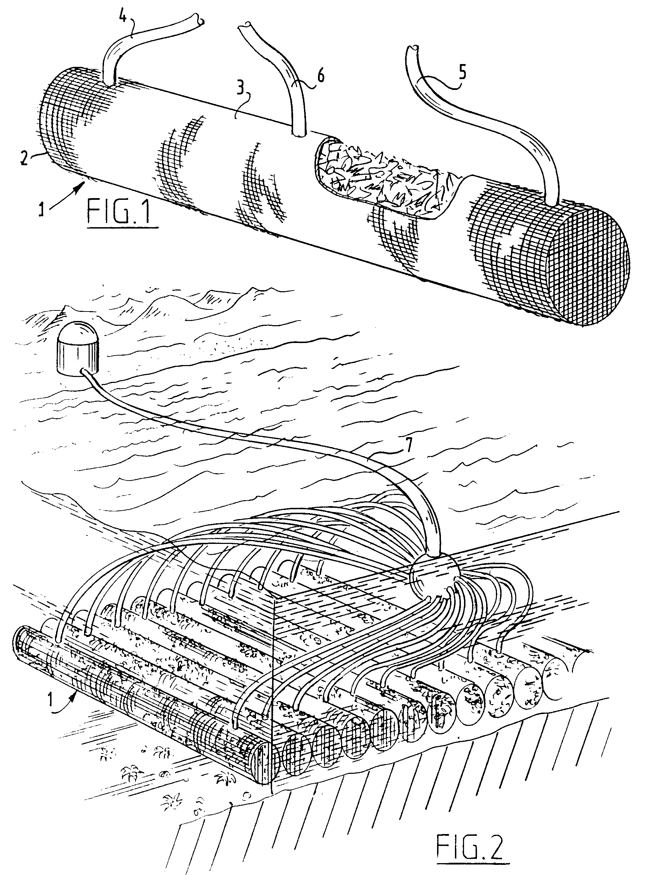

Figure 1 shows a container according to the invention, and
Figure 2 shows an assembly of containers to the invention.

A container 1 in which garbage is compressed consist of a web of metal wire 2 and a layer of sealing material 3, such as a foil of plastic for example. At the end parts of the container as well as in the middle part thereof flexible tubes 4, 5 and 6 are connected to the interior of the container. Gas by fermentation can be transported by the tubes to a place at a distance of the containers in order to be used, for example by burning. It is possible to connect the tubes to one single collecting tube 7 for transport of the gas to the location where the gas is being used.

In order to assure that the container will sink, both end parts of the container can be filled with heavy material, such as rock etc., if at all necessary.

## Claims

1. Method for processing garbage comprising the consecutive method steps:
- separating the garbage in fractions,
- compressing the fractions in a container according to a desired predetermined composition,
- placing the container in a casing consisting of a web of metal wire coated with plastic,
characterized by sealing said container with respect to the environment and connecting the container by means of a tube to a device for using fermentation gas.

2. Method according to claim 1, characterized in that at any of the end parts of the container a tube is connected.

3. Method according to claim 2, characterized by a tube connected to the middle part of the container.

4. Method according to claims 1-3, characterized in that said device for using fermentation gas is a gas burner for powering an electrical generator.
